# EUROPEAN PATENT APPLICATION

(11) **EP 2 578 190 A1**
(43) Date of publication of application: **10.04.2013**
(21) Application number: 11184410.6
(22) Date of filing: 07.10.2011
(51) Int. Cl.: A61F 9/00, A61M 37/00

(54) **Intraocular injection device**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is a medicament delivery device (1) comprising a needle (7) and a layer (9) of a composition coupled to at least a portion of the needle (7). The composition has a low viscosity and a low surface tension.

## Description

The present invention relates to a medicament delivery device, such as an apparatus for intraocular injection, and a needle for same.

### Background

A number of vision-threatening disorders or diseases of the eye need to deliver a medicament (pharmaceutical, biological, etc.) and/or implantable device to a posterior segment of the eye by intraocular delivery (more specifically intravitreal delivery) using a medicament delivery device. One such technique for intraocular delivery is accomplished by intraocular injection into the vitreous body.

A medicament delivery device (e.g., a syringe) may be used to administer therapeutic substances to eyes, such as eyes of mammals having eye disorders or diseases. The device may comprise a needle arrangement at its distal end which penetrates the eye for medicament delivery.

The programs of therapy offered to patients suffering from certain eye diseases and conditions often comprise delivery of medicament to the interior of the eye at regular intervals for long periods of time (perhaps months or even for life). It is therefore desirable to provide a medicament delivery device that works automatically. Such a device would provide benefits in terms of time, cost, dose accuracy, patient safety and pre requisite skill level on behalf of the heath care provider concerned.

When carrying out invasive procedures on the eye, e.g. administering medicaments into the vitreous body, it is essential that the surrounding area is rinsed and properly cleaned and disinfected. The current practice is to irrigate with saline and then disinfect with iodine solution. This involves number of steps and is subject to human error. Thus, there is a need for improvements.

### Summary

It is an object of the present invention to provide a medicament delivery device or a needle allowing to clean, disinfect and/or sterilize at least the punctured site automatically.

In an exemplary embodiment, a medicament delivery device according to the present invention comprises a needle and a layer of a composition coupled to at least a portion of the needle. The composition has a low viscosity and a low surface tension. The delivery device further comprises a barrel containing a medicament; and a plunger axially movable within the barrel.

In an exemplary embodiment, the layer does not cover a distal tip of the needle.

In an exemplary embodiment, the layer covers an entire exposed surface of the needle.

In an exemplary embodiment, the composition includes at least one of a disinfectant, a solution to promote healing, an analgesic, an antibiotic, a medicament, a marker and an antimicrobial.

In an exemplary embodiment, the layer deforms upon contact with an injection site.

The above mentioned advantages as well as other advantages of various aspects of the present invention will become apparent to those of ordinary skill in the art by reading the following detailed description, with appropriate reference to the accompanying drawings.

### Brief Description of the Drawings

Exemplary embodiments of the present invention are described herein with reference to the schematic drawings in which:
Figure 1 illustrates a perspective view of cross section of an exemplary embodiment of a medicament delivery device according to the invention prior puncturing an eye,
Figure 2 shows an enlarged sectional view of an exemplary embodiment of a medicament delivery device according to the invention,
Figure 3 depicts an exemplary embodiment of a medicament delivery device according to the invention in a perspective view of the cross section after puncturing the eye,
Figure 4 shows a perspective view of an exemplary embodiment of a medicament delivery device according to the invention, and
Figure 5 depicts an enlarged perspective view of an exemplary embodiment of a medicament delivery device according to the invention.

### Detailed Description

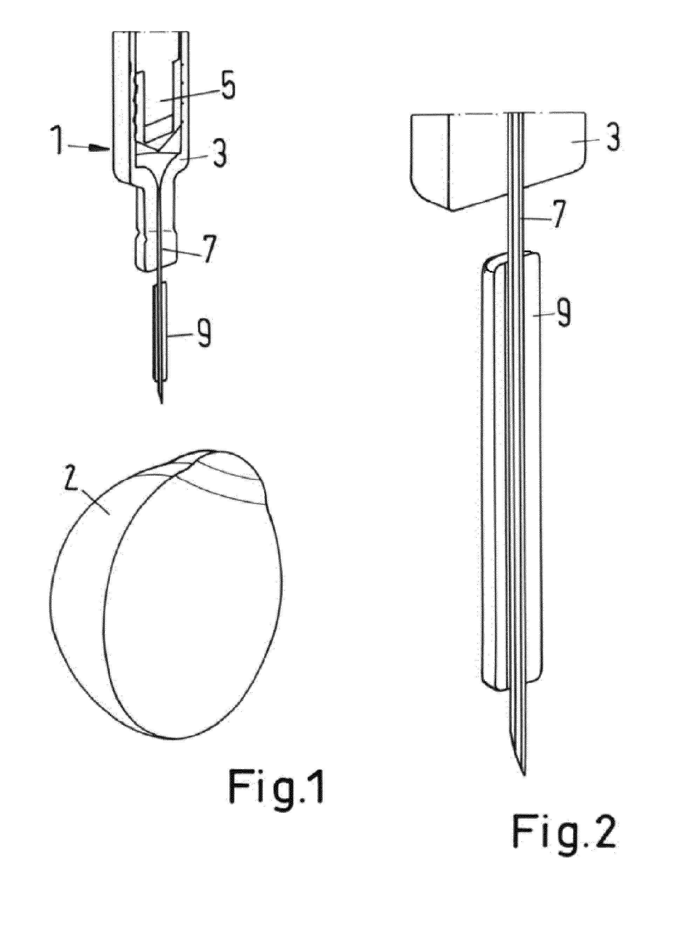

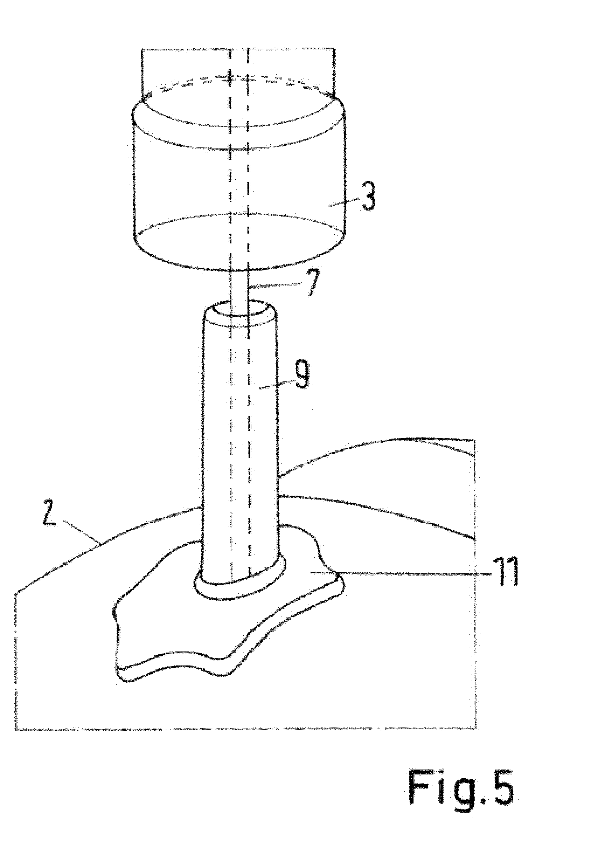

Figures 1 to 5 show an exemplary embodiment of a medicament delivery device 1. In the exemplary embodiment, the delivery device 1 may be a syringe which may be used alone or in conjunction with an auto-injector device. The delivery device 1 is shown having a generally cylindrical barrel 3. The barrel 3 a medicament or the like which is to be administered into the body part to be treated or diagnosed, e.g., an eye 2. Examples of such a medicament are steroids or monoclonal antibodies used to treat macula degeneration. Those of skilled in the art will understand that various medicament and/or therapeutic substances and/or implantable devices may be administered using the delivery device 1.

A plunger 5 is disposed within the barrel 3, and a needle 7 is coupled to a distal end of the barrel 3. When distally-directed pressure is applied to the plunger 5, the plunger 5 moves axially (distally) in the barrel 3 and expels the medicament through the needle 7. In an exemplary embodiment, the delivery device 1 may be an auto-injector, delivering the entire contents of the syringe when the apparatus is activated. In another exemplary embodiment, the apparatus may be a reusable, fixed dose delivery device for administering only a portion of the contents of the barrel 3 per injection. Those of skill in the art will understand that in another exemplary embodiment the delivery device 1 may be replaced by a medicament cartridge having a needle or having an interface for engaging a removable needle assembly.

In an exemplary embodiment, a layer 9 of a semi-solid composition is formed on at least a portion of an outer surface the needle 7. In an exemplary embodiment, the composition may be for the purpose of cleaning, disinfecting and/or sterilizing the injection site. For example, the composition may be a disinfectant, an antimicrobial, a solution to promote healing, etc. For example, the composition may be a cleaning and/or sterilizing agent to rinse and clean an injection site on the eye 11 prior to an injection. In addition or as an alternative, the composition may be a marker fluid, which contains a dye or granular pigmentation. Further, the composition may be colored so that the presence and location of the fluid 13 on the eye can be observed.

In an exemplary embodiment, the composition forming the layer 9 is a low viscosity gel which has a low surface tension but maintains its consistency and adherence to the needle 7 prior to use. In an exemplary embodiment, the delivery device 1 may be kept refrigerated prior to use to maintain the integrity of the layer 9.

In an exemplary embodiment, the layer 9 has the form of a hollow cylinder and encompasses at least a portion of the needle 7 lateral surface area. The person skilled in the art understands that other layer forms are possible (e.g., axial striping, rings, helical, conical, beaded, etc.). Additionally, various thicknesses of the layer 9 are possible.

In the exemplary embodiment depicted in Figures 1 and 2, the layer 9 does not cover a full length of the needle 7. For example, a distal tip of the needle 7 may not be covered by the layer 9. Hence, in this exemplary embodiment, the layer 9 comes into contact with the treated body part, e.g., the eye 2, after the needle 7 has punctured the body part.

In another embodiment, the layer 9 may extend beyond the distal tip of the needle 7 and be introduced to, e.g., the eye 2, prior to insertion of the needle 7.

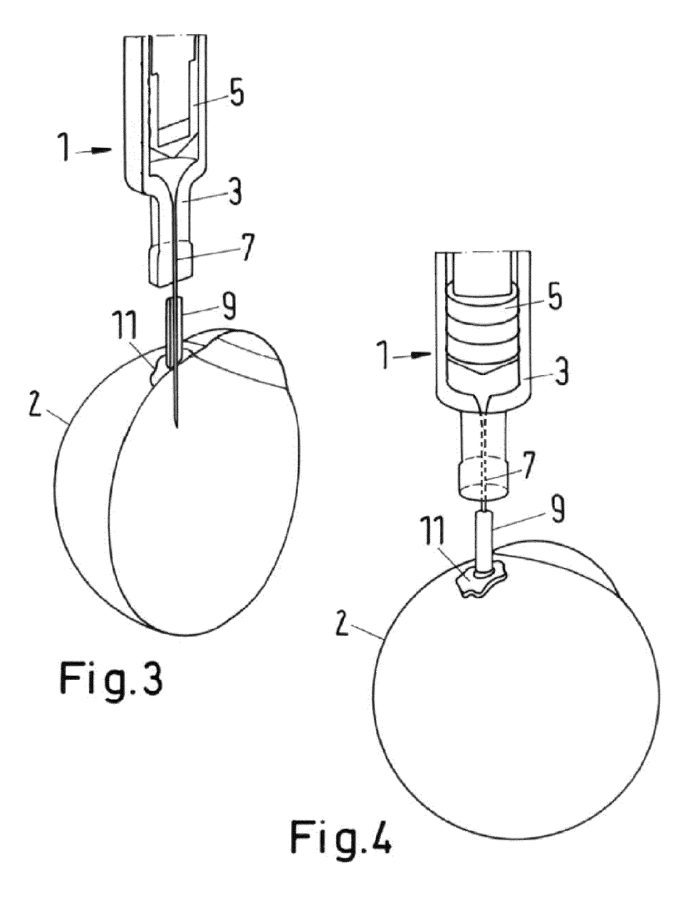

Figures 3 to 5 show the situation in which the needle 7 has punctured the eye 2 at a predetermined position, for example the limbus region of the eye 2.

Now, the needle 7 penetrates the eye 2 and advances within the eye 2 until it reaches the predefined depth within the eye. In an exemplary embodiment, in the moment in which the layer 9 reaches the surface of the eye 2, the surface tension of the layer 9 is breached and the integrity of the layer 9 is compromised, causing the composition to flow onto the surface of the eye 2 surrounding the site punctured by needle 7 forming a spot 11. Thereby, the composition is targeted and applied directly to the injection site, and the composition can clean, sterilize and/or disinfect the surface of the eye 2. Additionally, the composition may be absorbed into the eye 2 for further therapeutic effects.

In another exemplary embodiment, the needle 7 may be used with a safety needle assembly, such as those used with pen injectors.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A medicament delivery device (1) comprising:
a needle (7); and
a layer (9) of a composition coupled to at least a portion of the needle (7), wherein the composition has a low viscosity and a low surface tension.

2. The medicament delivery device (1) according to claim 1, further comprising:
a barrel (3) containing a medicament; and
a plunger (5) axially movable within the barrel (3).

3. The medicament delivery device (1) according to any one of the preceding claims, wherein the layer (9) does not cover a distal tip of the needle (7).

4. The medicament delivery device (1) according to any one of the preceding claims, wherein the layer (9) covers an entire exposed surface of the needle (7).

5. The medicament delivery device (1) according to any one of the preceding claims, wherein the composition includes at least one of a disinfectant, a solution to promote healing, an analgesic, an antibiotic, a medicament, a marker and an antimicrobial.

6. The medicament delivery device (1) according to any one of the preceding claims, wherein the layer (9) deforms upon contact with an injection site.
